# EUROPEAN PATENT SPECIFICATION

(11) **EP 1 792 565 B1**
(45) Date of publication and mention of the grant of the patent: **22.10.2008**
(21) Application number: 06255949.7
(22) Date of filing: 21.11.2006
(51) Int. Cl.: A61B 5/00

(54) **Selectively exposable miniature probes with integrated sensor arrays for continuous in vivo diagnostics**
Selektive Exposition von Miniatursonden mit integrierten Sensorarrays für kontinuierlichen in-vivo Diagnosis
L'exposition sélective d'une sonde miniature avec des reseaux de capteurs intégrés pour la diagnostique continue in-vivo

(30) Priority: 02.12.2005 US 293754
(43) Date of publication of application: 06.06.2007
(73) Proprietor: Cordis Corporation, Miami Lakes, FL 33014 (US)
(72) Inventor: Krulevitch, Peter, Pleasanton, CA 94566 (US); Noujaim, Sharbel, No. 25 Menlo Park, CA 94025 (US); Sieh, Zara, Pleasanton, CA 94566 (US)
(74) Representative: Gover, Richard Paul

(56) References cited:
- WO-A1-20/05048834
- US-A- 6 091 975
- US-A1- 2003 199 837
- US-B2- 6 551 838

## Description

The present invention relates to a medical device and more particularly to a medical detecting device comprising at least one miniature probe/lance (hereinafter microprotrusion) containing an integrated sensor which is covered with a protective coating to shield the sensor from fouling and other interactions with a host prior to its desired use. The protective coating is selectively removed from the sensor prior to using the same. In some embodiments of the present invention, some of the microprotrusions can include electrodes instead of sensors which can be used for electrical stimulation instead of sensing.

The ability to continuously sense biomolecules or other agents *in vivo* is highly desirable for the diagnosis, monitoring and treatment of diseases such as, for example, diabetes and cancer. However, despite decades of research and development, *in vivo* biosensors remain elusive due to four primary technical challenges, which include:

Fouling of the biosensor when implanted within the body which causes the sensor to drift, necessitating periodic calibration. Fouling is typically caused by accumulation of biomolecules other than those of interest at the sensor surface such as, for example, proteins, and bioencapsulation of the implant by the host's immune system.

Degradation and failure of the sensor resulting from interaction with the 'harsh' *in vivo* environment (e.g., elevated temperature and high ionic concentration).

Adverse response of the host's immune system (e.g., irritation/infection) to the implanted device.

The sensor and associated electronics must be small enough to implant comfortably within a body.

For the specific problem of monitoring glucose levels for the treatment of diabetes, several approaches have been taken. In the most commonly used approach, a small sample of blood (a few microliters) is extracted from the body and applied to a test strip that measures the glucose concentration electrochemically. Optical approaches also can be used to measure the glucose concentration of extracted blood. More recently, work has been done to measure glucose levels in extracted interstitial fluid (ISF), and to correlate the results with the blood glucose concentration. The advantage of ISF is the sample can be withdrawn from a shallow depth below the stratum corneum (i.e., the outer layer of the skin) reducing or eliminating the pain caused by lancing devices used to draw blood samples.

Approaches for *in vivo* measurement of glucose level in the blood and ISF include optical sensors implanted below the skin, electrochemical sensors implanted in tissue or within the blood vessels and direct near- or mid-IR measurement through the skin. Other than the direct near-IR measurement, which suffers from poor accuracy, all of the existing approaches suffer from the four issues mentioned above and are not currently practical for continuous monitoring over extended periods. Clinical trials are currently being conducted on electrochemical continuous glucose sensors implanted below the skin that work for about three days before they must be replaced.

US-6091975 discloses an agent detecting device comprising a plate having a plurality of microprotrusions for piercing the skin of a patient. Each of the microprotrusions has an electrode thereon for detecting the presence of an agent in the patient's interstitial fluid.

WO-2005/048834 discloses a long term analyte sensor for measuring an analyte in the body of a user, including a housing, a plurality of analyte contacting sensor elements and at least one structure for relaying information away from the sensor. The analyte contacting sensor elements are disposed in an array. At least one sensor protection membrane is provided that is controllable in a manner such that sensor elements may be activated at different times. The analyte may be glucose.

In view of the above, there is still a need for providing a new approach for continuously monitoring agents such as biomolecules that addresses all four of the primary technical challenges for *in vivo* biosensors.

The scope of the present invention is defined by the appended claims.

Embodiments of the present invention provides a medical detecting device that can be used for diagnosis, monitoring and/or treating a disease within a host in which a desired agent can be continuously sensed *in vivo* as well as a method of sensing, i.e., detecting, the selected agent utilizing the inventive medical detecting device. The inventive device can be used for diagnosis and monitoring of conditions of, for example, diabetes, cancer, congestive heart failure, cardiovascular disease, and neurological disorders.

Specifically, embodiments of the present invention provides a new approach to continuously sense a selected agent that addresses all four of the primary technical challenges for *in vivo* biosensors. The medical detecting device of the present invention may comprise at least one miniature microprotrusion that includes an integrated sensor thereon. Preferably, the medical detecting device of the present invention includes an array of such miniature microprotusions each including an integrated electrochemical sensor. Each sensor present within the inventive medical detecting device may be individually addressable, as described, for example, in US-6,091,975 to Daddona et al.,

In accordance with embodiments of the present invention, the inventive medical detecting device is applied to skin (human or animal) such that each microprotrusion extends through the stratum corneum, and makes contact with the interstitial fluid. The term "interstitial fluid" is used herein to denote one of the two components of extracellular fluid, the other being plasma. The interstitial fluid consists of a water solvent containing amino acids, sugars, fatty acids, coenzymes, hormones, neurotransmitters, salts as well as waster products from the cells. Plasma and interstitial fluid are essentially identical except for location. Plasma, the major component of blood, communicates freely with the interstitial fluid through pores and intercellular clefts in capillary endothelium. Blood pressure causes the continuous production of the interstitial fluid by pushing plasma through these openings into the interstitium. The pores are too small to allow proteins or cells through, but most other solutes can pass. Additionally, passive diffusion and active cellular transport from the blood also play a role in fluid production.

Each sensor may be covered with a biocompatible protective coating that shields the sensor from fouling and other interactions with the host when the individual sensor is not in use. To activate an individual sensor/sensors, the biocompatible protective coating may be selectively removed. The exposed sensor/sensors may then be used for a period of time, which is dictated by sensor drift. After exceeding the stable operation limit of the sensor/sensors, a new sensor/sensors may be selectively exposed and used for subsequent measurements. This process may be repeated until all sensors have been used, at which time the spent medical detecting device may be removed and a new one may be applied.

In general terms, the inventive medical detecting device may comprise a plate having at least one microprotrusion fixedly attached and extending from the plate; and at least one agent detecting sensor on the at least one microprotrusion, wherein said at least one agent detecting sensor is covered with a biocompatible protective coating which is selectively removed prior to detecting an agent, and said at least one microprotrusion having a length which locates the at least one agent detecting sensor below the outermost layer of a body surface and in contact with a body fluid.

In some embodiments of the present invention, some of the microprotrusions can include electrodes instead of sensors, which can be used for electrical stimulation instead of sensing. This embodiment allows for the medical device to include the use of electrical stimulation which can reduce the lag time between glucose levels in the blood and the interstitial fluid. As is known to those skilled in the art, the lag time can make it difficult to accurately determine how much insulin a diabetic patient should injection. It has been previously shown, in this regard, that electrical stimulation increases blood perfusion locally, which may cause glucose levels in the interstitial fluid to be closer to the glucose levels in the blood.

The medical detecting device described above, maybe used in a method of treatment, which comprises providing a medical detecting device that includes a plate having at least one microprotrusion fixedly attached and extending from the plate; and at least one agent detecting sensor on the at least one microprotrusion, wherein said at least one agent detecting sensor is covered with a biocompatible protective coating, and said at least one microprotrusion having a length which locates the at least one agent detecting sensor below the outermost layer of a body surface and in contact with a body fluid; selectively removing the biocompatible protective coating from the at least one agent detecting sensor to expose the sensor; and detecting an agent.

As stated above, some of the microprotrusions can include electrodes instead of sensors, which can be used for electrical stimulation instead of sensing. This embodiment includes the same basic processing steps as described above.

As stated above, a major advantage of the inventive medical detecting device and method of using the same is the shielding, i.e., protection, of a sensor to minimize fouling and other interactions from the host prior to the activation of the sensor. Another benefit of the inventive approach is that the sensors can be pre-calibrated at the factory and do not have to be recalibrated at any time during the measurement process. A still other benefit of the present invention, is that because the individual microprotrusions can be made very small (on the order of 100 µm (100 microns)) hundreds of sensors can be integrated into a single medical detecting device enabling agent, e.g., glucose, monitoring for extended periods before replacement. A further benefit of the present invention is that multiple sensors can be used simultaneously to obtain results with greater accuracy.

A still further benefit of the present invention is that the protective coating covering each sensor comprises a biocompatible material, which avoids an adverse host response. The term "biocompatible" is used herein to denote a material that is compatible with a living tissue or a living organism by not being toxic or injurious and by not causing immunological reaction.

In some embodiments of the present invention, the microprotrusions can also be coated with at least one drug which can be used to treat a disease, to minimize foreign-body responses, etc.

Other benefits of the present invention include: the inventive medical detecting device is convenient to apply and use; the sensors and the means for exposing them can be entirely electrically addressed enabling development of a system that is rugged, compact and comfortable to wear; a wireless communication chip can be incorporated within the inventive medical detecting device; the sensors do not have to survive extended periods while exposed to the *in vivo* environment; a simple, low cost, mass manufacturing approach can be used to form the same; and the inventive medical detecting device can be integrated with therapies and therapeutic devices for providing a closed-loop system.

The present invention, which provides a medical detecting device containing at least one microprotrusion including a sensor that is protected with a biocompatible protective coating prior to use thereof and a method of detecting an agent using the inventive medical detecting device, will now be described in greater detail by referring to the following discussion and drawings that accompany the present application. It is noted that the drawings of the present application are provided for illustrative purposes and, as such, they are not drawn to scale.

Before referring to the drawings that accompany the present application, it is noted that the drawings illustrate one embodiment of the present invention in which a plurality of microprotrusions are present and each protrusion contains a sensor that is covered with a biocompatible protective coating prior to using the medical detecting device. Although such a structure is illustrated, the present invention is not limited to any specific number of microprotrusions as long as the medical detecting device includes at least one of said microprotrusions.

The term "detecting" is used broadly herein to include detection of, or sensing the presence, or amount of an agent, as well as monitoring the presence, or amount of an agent. The term "agent" includes biomolecules, body electrolytes, alcohol, illicit drugs, pharmaceuticals, etc. that can be sampled through the skin. The major barrier properties of the skin, such as resistance to agent detecting, reside with the outer most layer (i.e., stratum corneum). The inner division of the epidermis generally comprises three layers commonly identified as stratum granulosum, stratum malpighii, and stratum germinativum. There is essentially little or no resistance to movement of an agent through the stratum granulosum, stratum malpighii, and stratum germinativum. The device of the present invention is used to pierce the stratum corneum for *in situ* detecting of an agent with an exposed sensor located below the outermost layer of the patient's skin.

Embodiments of the present invention will now be described by way of example with reference to the accompanying drawings, in which:
FIG. 1 is an enlarged perspective view of the bottom side of the inventive medical detecting device prior to use thereof including an array of microprotrusions with selectively exposable sensors; and
FIG. 2 is an enlarged perspective view of the structure shown in FIG. 1 after removing a protective coating from at least one of the sensors within the array.

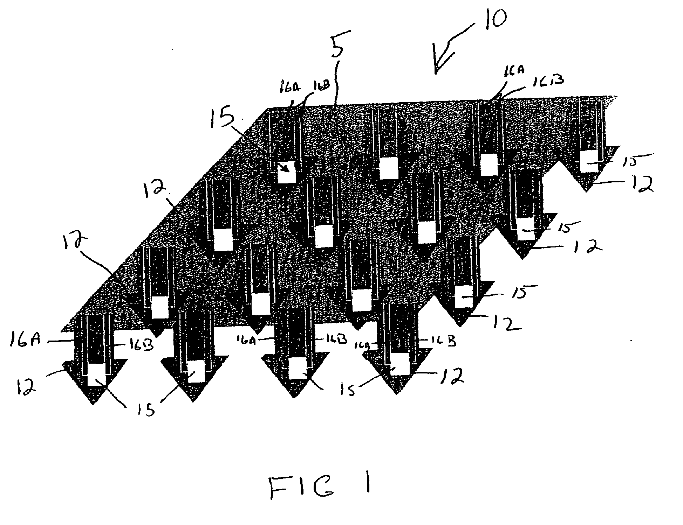

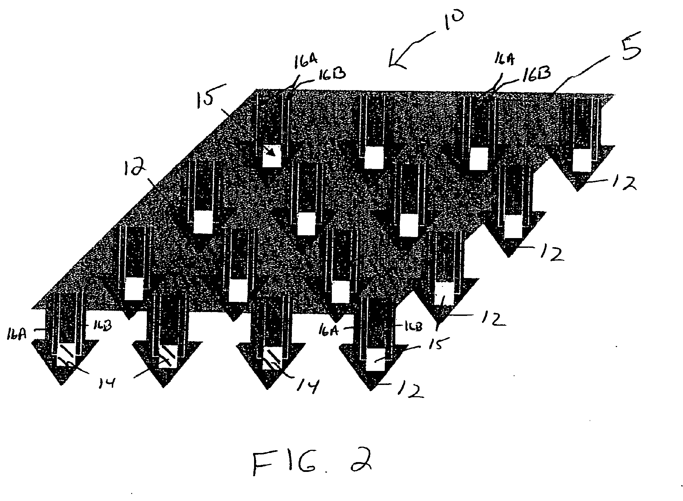

Reference is made to FIGS. 1 and 2, which illustrate a fragment 10 of a medical detecting device that can be used for piercing the skin; the device fragment 10 illustrated in FIG. 1 is prior to selectively removing the biocompatible protective coating 15 from at least one of the sensors, and the device fragment 10 in FIG. 2 is after removing the biocompatible protective coating 15 from at least one of the sensors. In FIG. 2, reference numeral 14 denotes an exposed agent-detecting sensor.

In some embodiments of the present invention, some of the microprotrusions can include electrodes (not specifically shown) instead of sensors, which can be used for electrical stimulation instead of sensing. This embodiment allows for the medical device to include the use of electrical stimulation which can reduce the lag time between glucose levels in the blood and the interstitial fluid. As is known to those skilled in the art, the lag time can make it difficult to accurately determine how much insulin a diabetic patient should injection. It has been previously shown, in this regard, that electrical stimulation increases blood perfusion locally, which may cause glucose levels in the interstitial fluid to be closer to the glucose levels in the blood.

The device fragment 10 includes a plurality of microprotrusions 12 which are sized and shaped for piercing the outermost stratum corneum layer of (e.g., human or other animal) skin. Each of the microprotrusions 12 extends outward from a surface of plate 5. As shown, each microprotrusion 12 includes a pair of conductive leads 16A and 16B that are in contact with a sensor present at the tip of each microprotrusion. In FIG. 1, each of the sensors is covered with a biocompatible protective coating 15. In FIG. 2, the biocompatible protective coating 15 is removed from at least one of the sensors providing an exposed sensor 14 that can be used for detecting a desired agent.

The device fragment 10 provides for the transcutaneous placement of at least one exposed sensor 14 (see FIG. 2) at a selected site within the body of a patient. Particularly, the device fragment 10 facilitates the placement of a flexible thin film electrochemical sensor of the type used for detecting specific parameters representative of patient conditions. Placing the exposed sensor 14 within the skin of the patient allows *in situ* readings to be obtained instead of relying on collecting interstitial fluid into an absorbing member. The *in situ* detection minimizes lag time in the readings compared to diagnostic methods, which rely on extracting the interstitial fluid before the measurement can take place. In one preferred embodiment, the exposed sensor 14 is designed to monitor glucose levels in diabetic patients.

The device fragment shown in FIG. 1 and 2 minus the biocompatible protective coating 15 is fabricated using techniques well known in the art. For example, the techniques described in US-6,091,975 to Daddona et al., can be used to manufacture the device fragment 10. Each sensor in the array is addressed in accordance with the disclosure of US-6,091,975. It is noted that the above is one example on how to fabricate the protrusions and the sensors that reside on the protrusions- other methods, sensors, and methods for making the protrusions and sensors are possible.

In accordance with the present invention, each of the microprotrusions 12 is sized appropriately so that they reach through the stratum corneum, but do not contact the patient's nerve endings. For example, the exposed sensors 14 at the tip of each microprotrusion 12 are about 100 micrometers in diameter and the microprotrusions 12 have an overall length of about 150 micrometers. With this configuration, the exposed sensor 14 is responsive to changes in the presence or amount of agent in the patient's interstitial fluid without causing a painful sensation or excessive bleeding. Each of the conductive leads present on the microprotrusions can be constructed using thin film mask techniques utilizing thin film conductors embedded or encased between layers of selected insulated material such as polyimide film. The exposed sensors 14 at the distal tip of each microprotrusion 12 are inserted into the patient's skin in order to contact the patient's interstitial fluid when the sensor is transcutaneously placed. The exposed sensors 14 are formed by techniques well known in the art including, for example, evaporation, electroplating, ink-jet spotting, printing, or pin spotting. The exposed sensor 14 includes any biosensor including, for example, electrochemical electrode sensors, fluorescence-based sensors, sensors based on binding of proteins, cells, or DNA, or mixtures and combinations thereof.

The microprotrusions 12 are generally formed from a single piece of material (although they need not be) and are sufficiently sharp and long for puncturing at least the stratum corneum of the body surface. In one embodiment, the microprotrusions 12 and the plate 5 are essentially impermeable or are impermeable to the passage of an agent. The width of each microprotrusion 12 can be any of a range of widths. Usually, the width of the microprotrusions 12 is in the range of about 25 micrometers to 500 micrometers. The length of the microprotrusions is subject to variation of the body surface being penetrated and corresponds to the natural thickness of the stratum corneum for one of the features of the invention is that the sensor detects the agent below the outermost layer of the epidermis. Usually, the microprotrusions 12 will be about 20 micrometers to about 400 micrometers in length. The microprotrusions 12 can have slanted (i.e., angled) leading edges to further reduce the insertion force required to press the microprotrusions 12 into the body surface. The leading edges of each microprotrusion can be all the same angle or can be at different angles suitable for piercing the body surface. Alternatively, the leading edge of each microprotrusions 12 can be arcuate (i.e., curved) in shape, having, for example, a convex or concave shape.

The pattern for any of the microprotrusion array can be produced with a photo-etching process. A thin plate 5 of metal such as stainless steel or titanium is etched photolithographically with patterns containing skin-piercing structures. In general, a thin laminate dry resist or wet resist is applied on the plate 5 which typically has a thickness of about 7 micrometers to about 100 micrometers, preferably about 25 micrometers to about 50 micrometers. The resist is contact exposed using a mask having the desired pattern and is subsequently developed. These operations are conducted in much the same way that they are for the manufacture of a printed circuit board. The plate 5 is then etched using acidic solutions. The sensors are formed on the sheet before the microprotrusions are bent out of plane. Standard photolithographic and printing techniques can be used to form the sensors on the flat sheet. When some of the sensors are replaced with electrodes for use in electrical stimulation, the electrodes are made utilizing the same procedures as the sensors. After the pattern has been etched through the plate, the plate 5 is placed on a die having a plurality of openings. A punch having a plurality of protrusions corresponding to any openings in the plate 6 and openings in the die is initially located above the plate and the die. At the initial stage, the microprotrusions 12 are in the same plane as the rest of the plate 5. The punch dies are then pressed into the openings, thus bending the microprotrusions 12 downward to be substantially perpendicular to the plane of the plate 5. The finished structure provides microprotrusions 12.

Generally, the microprotrusions 12 are at an angle of about 90 degrees to the surface of the plate 5 after being punched, but they can be disposed at any angle forward or backward from the perpendicular position that will facilitate penetration of and attachment to the body surface. In addition, other anchoring elements such as barbs, openings, etc. can be used with the angled microprotrusions 12 to further enhance anchoring of the device.

The plates 5 and microprotrusions 12 can be made from materials that have sufficient strength and manufacturability to produce microprotrusions 12, such as, rigid polymers, metals and metal alloys. Examples of metals and metal alloys include, but are not limited to: stainless steel, iron, steel, tin, zinc, silver, platinum, aluminum, zirconium, titanium and titanium alloys having nickel, molybdenum or chromium. Each of the plate 5 and microprotrusions 12 can have a thin layer of silver, gold, platinum, iridium, titanium, rhodium plating or evaporated or sputtered biocompatible metals to provide for inertness and biocompatibility. Examples of polymers include, but are not limited to: polystyrene, polymethylmethacrylate, polypropylene, phenolic resins such as that sold under the trade name "BAKELITE", cellulose acetate, ethyl cellulose, styrene/acrylonitrile copolymers, styrene/butadiene copolymers, acrylonitrile/butadiene/styrene (ABS) copolymers, polyvinyl chloride and acrylic acid polymers including polyacrylates and polymethacrylates.

In some embodiments of the present invention, the plate 5 itself can be used as one of the return leads.

The number of microprotrusions 12 and sensors is variable with respect to the redundancy desired in the system, the agent being detected, the type of sensor being used, the length of time that the device is intended to function, and other factors as will be evident to one of ordinary skill in the art.

In accordance with the present invention, and prior to use of at least one of the sensors, the sensors within the array are covered with a biocompatible protective coating 15, See FIG. 1. Suitable biocompatible protective coatings 15 that can be used in the present invention include, but are not limited to: metals such as, for example, Ti, NiTi, gold, platinum, and aluminum, and polymers such as, for example, silicone polymers (i.e., organosiloxanes), polyurethanes, polyamides, parylene, fluoropolymers such as, for example, Teflon, polyolefins such as, for example, polyethylene and polypropylene, collagen, chitin, alginate, polyvinyl pyrrolidone, polyethylene glycol, polyethylene oxide, polyvinyl alcohol, polyglycol lactic acid, polylactic acid, polycaprolactone, polyamino acid, and a hydrogel such as, for example, carboxymethyl cellulose. In one embodiment of the present invention, a biocompatible metal is used as the protective coating 15. In another embodiment of the present, a biocompatible polymer, particularly a silicone polymer, is used as the protective coating.

The protective coating 15 is applied to the sensor utilizing a conventional deposition process including, for example, spin-on coating, dip coating, spray coating, chemical vapor deposition, plasma enhanced chemical vapor deposition, plating, sputtering, evaporation, and other like deposition processes. Notwithstanding the deposition process used, the thickness of the protective layer 15 is typically from about 0.25 to about 20 µm (about 0.25 to about 20 microns), with a thickness from about 2 to about 5 µm (about 2 to about 5 microns) being more typical.

In some embodiments of the present invention, some of the microprotrusions 12 may also be coated with a drug for treating a specific medical condition or disease. The types of drugs that can be used in the present invention include, but are not limited to: antibiotics, antivirals, sirolimus, anticoagulants such as heparin, large molecule biopharmaceuticals, vaccines, hormones, and Natrecor. The drug coating is formed by incorporating a drug into a biocompatible material and then depositing the drug coating onto a specified microprotrusion 12. In some embodiments, the drug can be mixed within the protective coating described above. In other embodiments, the drug coating can be applied prior to, or after forming the protective coating on the sensors. The drug can be applied by dip-coating or rolling the liquid drug formulation onto the protrusions after they have been bent perpendicular to the plate. In this manner, the drug can be selectively released when removing the protective layer from specific protrusions, or the drug can elute at a steady rate from all microprotrusions that it is applied to.

During application, a specific sensor or a plurality of sensors are exposed by selectively removing the protective coating 15 from the desired sensors. In some applications in which electrodes for electrical stimulation are used in place of the sensors, the same procedure is followed. The protective coating 15 can be removed by thermal ablation, etching, electrically-activated dissolution, or electrically-activated corrosion.
US-6,551,838 to Santini, Jr. et al., US-6,849,463 to Santini, Jr. et al., and US-6,773,429 to Sheppard, Jr. et al. provide details concerning the electrically-activated dissolution and the electrically-activated corrosion processes that can be used in the present invention. In some embodiments, the protective coating 15 may be a biocompatible sheath that could be slid or rotated to expose a new sensor.

The exposed sensor 14 is then used for a period of time, which is dictated by sensor drift. After exceeding the stable operation limit of the sensor, a new sensor is selectively exposed, as described above, and used for subsequent measurements. This process is repeated until all sensors have been used, at which time the spent medical detecting device is removed and a new one is applied.

The device fragment 10 can optionally be made to adhere to the patient's body surface by various means, including an adhesive applied to the body-contacting side of plate 5 or other anchoring elements on the device fragment 10. Further, a watch band or elastic bandage can be used to maintain the device in contact with the skin. The adhesive should have sufficient tack to insure that the device 10 remains in place on the body surface during normal user activity, and yet permits reasonable removal after the predetermined (e.g., 24-hour) wear period. A suitable release liner (not shown) is preferably provided for maintaining the integrity of the adhesive before use. In use, the release liner is stripped from the adhesive before the device is applied to the skin.

The device fragment 10 of the present invention can also be used with fluid-attracting regimes including, but not limited to: reverse electrotransport (i.e., iontophoresis and/or electroosmosis), osmosis, and passive diffusion. Osmotic devices can be used to draw fluid from the body (i.e., interstitial fluid or sweat) which carries the agent to be detected, for example, reference may be had to US-4,756,314 of which the disclosed osmotic configurations can be used with the present invention. The osmotic device can be attached to a body surface by means of a flexible adhesive overlay.

The device fragment 10 can be interfaced with an electronic control unit (not shown) or external sensor (not shown). The electronics control unit can be attached to the device fragment 10, or can be a separate unit, or a combination of the two. The interfacing typically occurs along the upper surface of plate 5 using metallic traces which connect the interfaced device with the device fragment 10 described in the present application. Alternatively, the electronic interfacing can be accomplished using a wireless means.

In a preferred embodiment of the present invention, the inventive device is used over a prolonged period of time for periodically or continuously detecting a body parameter such as, for example, glucose, in a diabetic patient. Such readings are useful in monitoring the patient's blood glucose concentration (i.e., through appropriate software which correlates the concentration of glucose in the blood) and can be further used to adjust the treatment regime which includes administration of insulin to the patient and/or appropriate modification of diet and/or exercise. In some embodiments of the present invention, the insulin may be contained within a coating found on at least one of the microprotrusions.

The inventive device and method described above can be easily integrated into other *in vivo* applications, including but not limited to: transdermal diagnostics, implanted medical devices, catheter-based systems for minimally invasive procedures and endoscope-based systems.

## Claims

1. A medical detecting and stimulating device comprising:
a plate (5) having at least one microprotrusion (12) fixed attached and extending from the plate (5);
at least one agent detecting sensor (14) on the at least one microprotrusion (12), wherein said at least one agent detecting sensor (14) is covered with a biocompatible protective coating (15) which can be selectively removed prior to detecting an agent, and said at least one microprotrusion (12) having a length which is arranged to locate the at least one agent detecting sensor (14) below the outermost layer of a body surface and in contact with a body fluid; and
at least one microprotrusion (12) including an electrode operable to provide electrical stimulation.

2. The medical detecting and stimulating device of Claim 1 further comprises other agent detecting sensors (14) that are exposed.

3. The medical detecting and stimulating device of Claim 1 wherein said at least one agent detecting sensor (14) has a pair of conductive leads (16A, 16B) that extend to a surface of said plate (5).

4. The medical detecting and stimulating device of Claim 1 wherein said at least one agent detecting sensor (14) is a biosensor.

5. The medical detecting and stimulating device of Claim 4 wherein said biosensor comprises an electrochemical electrode sensor.

6. The medical detecting and stimulating device of Claim 1 further comprising a coating containing a drug for treating a medical conditions covering at least one of said agent detecting sensors (14).

7. The medical detecting and stimulating device of Claim 1 wherein said plate (5) is interfaced with an external electrical control unit or sensor.

8. The medical detecting and stimulating device of Claim 1 wherein said biocompatible protective coating (15) comprises a metal or a polymer.

9. The medical detecting and stimulating device of Claim 8 wherein said metal comprises one of Ti, NiTi, gold, platinum or aluminum.

10. The medical detecting and stimulating device of Claim 8 wherein said polymer comprises one of silicone polymers, polyurethanes, polyamides, parylene, fluoropolymers, polyolefins, collagen, chitin, alginate, polyvinyl pyrrolidone, polyethylene glycol, polyethylene oxide, polyvinyl alcohol, polyglycol lactic acid, polylactic acid, polycaprolactone, polyamino acid, or hydrogels.

11. The medical detecting and stimulating device of Claim 1 wherein said body fluid is interstitial fluid.

12. The medical detecting and stimulating device of Claim 1 wherein said plate (5) serves as a return lead.

13. The medical detecting and stimulating device of Claim 1 wherein said agent detecting sensor (14) is operable to detect a biomolecule, a body electrolyte, alcohol, an illicit drug, or a pharmaceutical.

14. The medical detecting and stimulating device of Claim 1 wherein said agent detecting sensor (14) is operable to detect glucose.

## Patentansprüche

1. Medizinische Vorrichtung zum Detektieren und Stimulieren, die Folgendes umfasst:
eine Platte (5) mit wenigstens einem Mikrovorsprung (12), der fest an der Platte (5) angebracht ist und sich von dieser erstreckt;
wenigstens einen Agens detektierenden Sensor (14) an dem wenigstens einen Mikrovorsprung (12), wobei der wenigstens eine Agens detektierende Sensor (14) mit einer bioverträglichen Schutzbeschichtung (15) bedeckt ist, die selektiv entfernt werden kann vor dem Detektieren einer Agens, und wobei der wenigstens einen Mikrovorsprung (12) eine Länge aufweist, die eingerichtet ist, um den wenigstens einen Agens detektierenden Sensor (14) unter der äußersten Schicht einer Körperoberfläche und in Kontakt mit einem Körperfluid in Position zu bringen; und
wobei der wenigstens ein Mikrovorsprung (12) eine Elektrode umfasst, die in der Lage ist, für eine elektrische Stimulation zu sorgen.

2. Medizinische Vorrichtung zum Detektieren und Stimulieren nach Anspruch 1, die weiterhin andere Agens detektierende Sensoren (12) umfasst, die aufgedeckt sind.

3. Medizinische Vorrichtung zum Detektieren und Stimulieren nach Anspruch 1, bei welcher der wenigstens eine Agens detektierende Sensor (12) ein Paar von Leitungen aufweist, die sich zu der Oberfläche der Platte (5) erstrecken.

4. Medizinische Vorrichtung zum Detektieren und Stimulieren nach Anspruch 1, bei welcher der wenigstens eine Agens detektierende Sensor (12) ein Biosensor ist.

5. Medizinische Vorrichtung zum Detektieren und Stimulieren nach Anspruch 4, bei welcher der Biosensor einen elektrochemischen Elektrodensensor umfasst.

6. Medizinische Vorrichtung zum Detektieren und Stimulieren nach Anspruch 1, die weiterhin eine Beschichtung umfasst, die einen Wirkstoff zum Behandeln eines medizinischen Zustandes enthält, und die wenigstens einen der Agens detektierenden Sensoren (14) bedeckt.

7. Medizinische Vorrichtung zum Detektieren und Stimulieren nach Anspruch 1, bei welcher die Platte (5) mit einer externen elektrischen Steuereinheit oder einem Sensor in Verbindung steht.

8. Medizinische Vorrichtung zum Detektieren und Stimulieren nach Anspruch 1, bei welcher die bioverträgliche Schutzbeschichtung (15) ein Metall oder ein Polymer umfasst.

9. Medizinische Vorrichtung zum Detektieren und Stimulieren nach Anspruch 8, bei welcher das Metall Ti, NiTi, Gold, Platin oder Aluminium umfasst.

10. Medizinische Vorrichtung zum Detektieren und Stimulieren nach Anspruch 8, bei welcher das Polymer Silikonpolymere, Polyurethane, Polyamide, Parylen, Fluoroplymere, Polyolefine, Kollagen, Chitin, Alginat, Polyvinylpyrrolidon, Polyethylenglykol, Polyethylenoxide, Polyvinylalkohol, Polyglykolmilchsäure, Polymilchsäure, Plycaprolakton, Polyaminosäure oder Hydrogele umfasst.

11. Medizinische Vorrichtung zum Detektieren und Stimulieren nach Anspruch 1, bei welcher das Körperfluid ein interstitielles Fluid ist.

12. Medizinische Vorrichtung zum Detektieren und Stimulieren nach Anspruch 1, bei welcher die Platte (5) als ein Rückleiter dient.

13. Medizinische Vorrichtung zum Detektieren und Stimulieren nach Anspruch 1, bei welcher der Agens detektierende Sensor (14) dazu in der Lage ist, ein Biomolekül, einen Körperelektrolyten, Alkohol, einen illegalen Wirkstoff oder ein Pharmazeutikum zu detektieren.

14. Medizinische Vorrichtung zum Detektieren und Stimulieren nach Anspruch 1, bei welcher der Agens detektierende Sensor (14) in der Lage ist, Glukose zu detektieren.

## Revendications

1. Dispositif médical de détection et de stimulation comprenant :
◆ une plaque (5) comprenant au moins une microprotubérance (12) fixée et s'étendant depuis la plaque (5) ;
◆ au moins un capteur de détection d'agent (14) sur ladite microprotubérance (12), dans lequel ledit au moins un capteur de détection d'agent (14) est recouvert d'une couche de protection biocompatible (15) apte à être retirée de façon sélective avant la détection d'un agent, et ledit au moins une microprotubérance (12) ayant une longueur dans laquelle est prévue l'au moins un capteur de détection d'agent (14) sous la couche externe d'une surface corporelle et en contact avec un fluide corporel ; et
◆ au moins une microprotubérance (12) comprenant une électrode apte à provoquer une stimulation électrique.

2. Dispositif médical de détection et de stimulation selon la revendication 1, comprenant en outre d'autres capteurs de détection d'agent (14) qui sont exposés.

3. Dispositif médical de détection et de stimulation selon la revendication 1, dans lequel ledit au moins un capteur de détection d'agent (14) comprend deux plombs conducteurs (16A, 16B) s'étendant sur une surface de ladite plaque (5).

4. Dispositif médical de détection et de stimulation selon la revendication 1, dans lequel ledit au moins un capteur de détection d'agent (14) est un biocapteur.

5. Dispositif médical de détection et de stimulation selon la revendication 4, dans lequel ledit biocapteur comprend un capteur électrochimique à électrode.

6. Dispositif médical de détection et de stimulation selon la revendication 1, comprenant en outre un enrobage contenant un médicament destiné au traitement médical et recouvrant au moins l'un desdits capteurs de détection d'agent (14).

7. Dispositif médical de détection et de stimulation selon la revendication 1, dans lequel ladite plaque (5) est relié à une unité de commande électrique ou un capteur externe.

8. Dispositif médical de détection et de stimulation selon la revendication 1, dans lequel ladite couche de protection biocompatible (15) comprend un métal ou un polymère.

9. Dispositif médical de détection et de stimulation selon la revendication 8, dans lequel ledit métal comprend l'un parmi du Ti, du NiTi, de l'or, du platine ou de l'aluminium.

10. Dispositif médical de détection et de stimulation selon la revendication 8, dans lequel ledit polymère comprend l'un parmi des polymères de silicone, des polyuréthanes, des polyamides, du parylène, des fluoropolymères, des polyoléfines, du collagène, de la chitine, de l'alginate, de la pyrrolidone de polyvinyle, du polyéthylène glycol, de l'oxyde de polyéthylène, de l'alcool de polyvinyle, de l'acide lactique polyglycolique, de l'acide polylactique, du polycaprolactone, du polyamino-acide ou des hydrogels.

11. Dispositif médical de détection et de stimulation selon la revendication 1, dans lequel ledit fluide corporel est un fluide interstitiel.

12. Dispositif médical de détection et de stimulation selon la revendication 1, dans lequel ladite plaque (5) sert de conducteur de retour.

13. Dispositif médical de détection et de stimulation selon la revendication 1, dans lequel ledit capteur de détection d'agent (14) est apte à détecter une biomolécule, un électrolyte corporel, de l'alcool, une drogue illicite ou un produit pharmaceutique.

14. Dispositif médical de détection et de stimulation selon la revendication 1, dans lequel ledit capteur de détection d'agent (14) est apte à détecter du glucose.
